# EUROPEAN PATENT SPECIFICATION

(11) **EP 2 771 685 B1**
(45) Date of publication and mention of the grant of the patent: **13.06.2018**
(21) Application number: 12843478.4
(22) Date of filing: 24.10.2012
(51) Int. Cl.: G01N 33/50, C12Q 1/68, A61P 35/00

(54) **BIOMARKERS OF CANCER**
KREBSBIOMARKER
BIOMARQUEURS DU CANCER

(30) Priority: 24.10.2011 US 201161550617 P
(43) Date of publication of application: 03.09.2014
(73) Proprietor: The General Hospital Corporation, Boston, MA 02114 (US)
(72) Inventor: TING, David Tsai, Dover, Massachusetts 02030 (US); MAHESWARAN, Shyamala, Lexington, Massachusetts 02421 (US); HABER, Daniel A., Chestnut Hill, Massachusetts 02467 (US)
(74) Representative: Boult Wade Tennant
(86) International application number: PCT/US2012/061576
(87) International publication number: WO 2013/063035

(56) References cited:
- EP-A1- 1 961 825
- WO-A1-2006/108048
- WO-A1-2011/127219
- WO-A2-2004/031412
- US-A1- 2002 042 072
- D. T. TING ET AL: "Aberrant Overexpression of Satellite Repeats in Pancreatic and Other Epithelial Cancers", SCIENCE, vol. 331, no. 6017, 4 February 2011 (2011-02-04), pages 593-596, XP055085541, ISSN: 0036-8075, DOI: 10.1126/science.1200801 -& D. T. TING ET AL: "SUPPORTING ONLINE MATERIAL: Aberrant Overexpression of Satellite Repeats in Pancreatic and Other Epithelial Cancers", SCIENCE, vol. 331, no. 6017, 13 January 2011 (2011-01-13), pages 593-596, XP055191527, ISSN: 0036-8075, DOI: 10.1126/science.1200801
- EWAN A GIBB ET AL: "The functional role of long non-coding RNA in human carcinomas", MOLECULAR CANCER, BIOMED CENTRAL, LONDON, GB, vol. 10, no. 1, 13 April 2011 (2011-04-13) , page 38, XP021097867, ISSN: 1476-4598, DOI: 10.1186/1476-4598-10-38
- EWAN A. GIBB ET AL: "Human Cancer Long Non-Coding RNA Transcriptomes", PLOS ONE, vol. 6, no. 10, 3 October 2011 (2011-10-03), page e25915, XP055191545, DOI: 10.1371/journal.pone.0025915
- CHEN ET AL: "The HSP90 family of genes in the human genome: Insights into their divergence and evolution", GENOMICS, ACADEMIC PRESS, SAN DIEGO, US, vol. 86, no. 6, 1 December 2005 (2005-12-01), pages 627-637, XP005265827, ISSN: 0888-7543, DOI: 10.1016/J.YGENO.2005.08.012

## Description

### TECHNICAL FIELD

Methods for diagnosing cancer and monitoring treatment efficacy based on detecting the presence of increased levels of expression of satellite correlated genes.

### BACKGROUND

Genome-wide sequencing approaches have revealed an increasing set of transcribed non-coding sequences, including "pervasive transcription" by heterochromatic regions of the genome linked to transcriptional silencing and chromosomal integrity (J. Berretta, A. Morillon, EMBO Rep 10, 973 (Sep, 2009); A. Jacquier, Nat Rev Genet 10, 833 (Dec, 2009)). In the mouse, heterochromatin is comprised of centric (minor) and pericentric (major) satellite repeats that are required for formation of the mitotic spindle complex and faithful chromosome segregation (M. Guenatri, D. Bailly, C. Maison, G. Almouzni, J Cell Biol 166, 493 (Aug 16, 2004)), whereas human satellite repeats have been divided into multiple classes with similar functions (J. Jurka et al., Cytogenet Genome Res 110, 462 (2005)). Bidirectional transcription of satellites in yeast maintains silencing of centromeric DNA through the Dicer mediated RNA-induced transcriptional silencing (RITS) and through a recently identified Dicer-independent pathway (M. Halic, D. Moazed, Cell 140, 504 (Feb 19)), although centromeric satellite silencing mechanisms in mammals are less well defined (A. A. Aravin, G. J. Hannon, J. Brennecke, Science 318, 761 (Nov 2, 2007)). Accumulation of satellite transcripts in mouse and human cell lines results from defects in *DICER1* (C. Kanellopoulou et al., Genes Dev 19, 489 (Feb 15, 2005); T. Fukagawa et al., Nat Cell Biol 6, 784 (Aug, 2004)) and from DNA demethylation, heat shock, or the induction of apoptosis (H. Bouzinba-Segard, A. Guais, C. Francastel, Proc Natl Acad Sci USA 103, 8709 (Jun 6, 2006); R. Valgardsdottir et al., Nucleic Acids Res 36, 423 (Feb, 2008)). Stress-induced transcription of satellites in cultured cells has also been linked to the activation of retroelements encoding RNApolymerase activity such as LINE-1 (D. Ugarkovic, EMBO Rep 6, 1035 (Nov, 2005); D. M. Carone et al., Chromosoma 118, 113 (Feb, 2009)). Despite these *in vitro* models, the global expression of repetitive ncRNAs in primary tumors has not been analyzed, due to the bias of microarray platforms toward annotated coding sequences and the specific exclusion of repeat sequences from standard analytic programs.

Using digital gene expression analysis, it has been shown that noncoding RNAs transcribed from satellite repeats are overexpressed in mouse and human epithelial cancers (Ting et al., Science 331, 593 (Feb 2011)).

### SUMMARY

The present invention is based, at least in part, on the identification of massive expression of satellite repeats in tumor cells, and of increased levels of satellite correlated genes, e.g., in tumor cells including circulating tumor cells (CTCs). Described herein are methods for diagnosing cancer, e.g., solid malignancies of epithelial origin such as pancreatic, lung, breast, prostate, renal, ovarian or colon cancer, based on the presence of increased levels of those satellite correlated genes.

The invention provides an *in vitro* method of detecting the presence of pancreatic cancer in a subject, the method comprising:
determining an expression level of HSP90BB (heat shock protein 90kDa alpha (cytosolic), class B member 2, pseudogene (HSP90AB2P)) in a sample comprising a test cell from the subject to obtain a test value; and
comparing the test value to a reference value,
wherein a test value that is significantly above the reference value indicates that the subject has pancreatic cancer.

Described herein are *in vitro* methods of detecting the presence of cancer in a subject. The methods include determining an expression level of one or more Satellite Correlated Genes selected from the group consisting of HSP90BB (heat shock protein 90kDa alpha (cytosolic), class B member 2, pseudogene (HSP90AB2P)); NR_003133 (Homo sapiens guanylate binding protein 1, interferon-inducible pseudogene 1 (GBP1P1), non-coding RNA); BX649144 (Tubulin tyrosine ligase (TTL)); DERP7 (transmembrane protein 45A (TMEM45A)); MGC4836 (Homo sapiens similar to hypothetical protein (L1H 3 region)); BC037952 (cDNA clone); AK056558 (cDNA clone); NM_001001704 (FLJ44796 hypothetical); ODF2L (outer dense fiber of sperm tails 2-like (ODF2L)); BC041426 (C12orf55 chromosome 12 open reading frame 55 (C12orf55)); )REXO1L1 (RNA exonuclease 1 homolog (S. cerevisiae)-like 1(REXO1L1)); AK026100 (FLJ22447 hypothetical LOC400221(FLJ22447)); AK026825 (transmembrane protein 212 (TMEM212)); KENAE1 (Homo sapiens mRNA for Kenae1 (AB024691)); HESRG (ESRG hypothetical LOC790952 (ESRG)); AK095450 (LOC285540 hypothetical LOC285540); FLJ36492 (CCR4-NOT transcription complex, subunit 1 (CNOT1)); AK124194 (FLJ42200 protein); AK096196 (hypothetical LOCI00129434); AK131313 (Zinc finger protein 91 pseudogene (LOC441666)); FLJ11292 (hypothetical protein FLJ11292); CCDC122 (coiled-coil domain containing 122 (CCDC122)); and BC070093 (cDNA clone) in a sample comprising a test cell from the subject to obtain a test value; and comparing the test value to a reference value. A test value that is significantly above the reference value indicates that the subject has cancer.

In some embodiments, the reference level is a level of the Satellite Correlated Gene in a normal cell. In some embodiments, the normal cell is a cell of the same type as the test cell in the same subject. In some embodiments, the normal cell is a cell of the same type as the test cell in a subject who does not have cancer. In some embodiments, the cell is in a tissue sample.

In some embodiments, the sample is known or suspected to comprise tumor cells, e.g., a blood sample known or suspected of comprising circulating tumor cells (CTCs), or a biopsy sample known or suspected of comprising tumor cells.

In some embodiments, the methods further include diagnosing a subject with cancer based on the presence of a test value that is significantly above the reference value; identifying the subject as having cancer based on the presence of a test value that is significantly above the reference value; selecting a subject for treatment based on the presence of a test value that is significantly above the reference value; or selecting a subject for further diagnostic testing (e.g., imaging, biopsy, etc) based on the presence of a test value that is significantly above the reference value.

The invention provides an *in vitro* method of evaluating the efficacy of a treatment for pancreatic cancer in a subject, the method comprising:
determining a level of HSP90BB in a first sample from the subject to obtain a first value;
determining a level of HSP90BB in a subsequent sample obtained from the subject at a later time after the treatment, to obtain a treatment value; and
comparing the first value to the treatment value,
wherein a treatment value that is below the first value indicates that the treatment is effective.

Described herein are *in vitro* methods for evaluating the efficacy of a treatment for cancer in a subject. The methods include determining a level of one or more Satellite Correlated Genes selected from the group consisting of HSP90BB (heat shock protein 90kDa alpha (cytosolic), class B member 2, pseudogene (HSP90AB2P)); NR_003133 (Homo sapiens guanylate binding protein 1, interferon-inducible pseudogene 1 (GBP1P1), non-coding RNA); BX649144 (Tubulin tyrosine ligase (TTL)); DERP7 (transmembrane protein 45A (TMEM45A)); MGC4836 (Homo sapiens similar to hypothetical protein (L1H 3 region)); BC037952 (cDNA clone); AK056558 (cDNA clone); NM_001001704 (FLJ44796 hypothetical); ODF2L (outer dense fiber of sperm tails 2-like (ODF2L)); BC041426 (C12orf55 chromosome 12 open reading frame 55 (C12orf55)); )REXO1L1 (RNA exonuclease 1 homolog (S. cerevisiae)-like 1(REXO1L1)); AK026100 (FLJ22447 hypothetical LOC400221(FLJ22447)); AK026825 (transmembrane protein 212 (TMEM212)); KENAE1 (Homo sapiens mRNA for Kenae1 (AB024691)); HESRG (ESRG hypothetical LOC790952 (ESRG)); AK095450 (LOC285540 hypothetical LOC285540); FLJ36492 (CCR4-NOT transcription complex, subunit 1 (CNOT1)); AK124194 (FLJ42200 protein); AK096196 (hypothetical LOCI00129434); AK131313 (Zinc finger protein 91 pseudogene (LOC441666)); FLJ11292 (hypothetical protein FLJ11292); CCDC122 (coiled-coil domain containing 122 (CCDC122)); and BC070093 (cDNA clone) in a first sample from the subject to obtain a first value; administering a treatment for cancer to the subject; determining a level of the one or more (i.e., the same) Satellite Correlated Genes in a subsequent sample obtained from the subject at a later time, to obtain a treatment value; and comparing the first value to the treatment value. A treatment value that is below the first value indicates that the treatment was effective (no change, or a decrease, means the treatment was ineffective).

In some embodiments, the first and second samples are known or suspected to comprise tumor cells, e.g., blood samples known or suspected of comprising circulating tumor cells (CTCs), or biopsy samples known or suspected of comprising tumor cells.

In some embodiments, the treatment includes administration of a surgical intervention, chemotherapy, radiation therapy, or a combination thereof.

In some embodiments of the methods described herein, the subject is a human.

In the methods of the invention the cancer is pancreatic cancer.

In some embodiments of the methods described herein, the methods include determining a level of one or more, e.g., two, three, or four, of AK056558; BC037952; HSP90BB; and/or AK096196. In some embodiments of the methods described herein, the methods include determining a level of one or both of HSP90BB and/or AK056558. In the methods of the invention, the methods include determining a level of HSP90BB. In some embodiments, the methods include determining a level of AK096196. In some embodiments, the methods include determining a level of AK056558. In some embodiments, the methods include determining a level of BC037952.

In some embodiments, determining a level of one or more Satellite Correlated Genes comprises determining a level of a transcript. In some embodiments, determining a level of a transcript comprises contacting the sample with an oligonucleotide probe that binds specifically to the transcript. In some embodiments, the probe is labeled.

In some embodiments, "determining a level" comprises detecting the presence or absence, e.g., the presence of a level above the limit of detection of the assay being used.

In some embodiments, the present methods can be used for determining the likelihood that a subject has cancer.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting.

Other features and advantages of the invention will be apparent from the following detailed description and figures, and from the claims.

### DESCRIPTION OF DRAWINGS

FIG. 1A is a bar graph showing levels of major satellite in percent of all genomic aligned reads among different tumors, cell lines, and tissues. Genotype of primary tumors and cell lines indicated below each tumor type and cell line. (Kras = KrasG12D; Tp53, SMAD4, and APC represent genes deleted)
FIG. 1B is a graphical representation of sequence read contributions from major satellite among all primary tumors, cancer cell lines, and normal tissues.
FIG. 2A shows the results of Northern blot analysis of three KrasG12D, Tp531ox/+ pancreatic primary tumors (Tumors 1-3) and a stable cell line (CL3) derived from Tumor 3.
FIG. 2B shows the results of Northern blot analysis of CL3 before (0) and after (+) treatment with the DNA hypomethylating agent 5-azacitadine (AZA).
FIG. 2C shows the results of Northern blot analysis of total RNA from multiple adult and fetal mouse tissues. All Northern blots exposed for approximately 30 minutes.
FIG. 2D is a pair of photomicrographs showing the results of RNA in-situ hybridization (ISH) of normal pancreas (left) and primary pancreatic ductal adenocarcinoma (right), hybridized with a 1 kb major satellite repeat probe.
FIG. 2E is a set of three photomicrographs showing the results of ISH analysis of preneoplastic PanIN (P) lesion, adjacent to PDAC (T) and normal pancreas (N), showing positive staining in PanIN, with increased expression in full carcinoma. Higher magnification (40x) of PanIN (left) and PDAC (right) lesions.
FIG. 2F is a set of three photomicrographs showing marked expression of satellites in PDAC cells metastatic to liver, which itself does not express satellites (left). Large, glandular metastatic tumor deposits are readily identified by standard histological evaluation and stain for satellite (middle). Satellite ISH is sensitive enough to detect micrometastases in liver parenchyma not easily appreciated by standard histological analysis (right; arrowheads). All images at 20x magnification (scale bar = 100 µm).
FIG. 3A is a bar graph showing the Total satellite expression in human pancreatic ductal adenocarcinoma (PDAC), normal pancreas, other cancers (L - lung, K - kidney, O - ovary, P - prostate), and other normal human tissues (1 - fetal brain, 2 - brain, 3 - colon, 4 - fetal liver, 5 - liver, 6 - lung, 7 - kidney, 8 - placenta, 9 - prostate, and 10 - uterus) quantitated by DGE. Satellite expression is shown as transcripts per million aligned to human genome.
FIG. 3B is a bar graph showing a breakdown of satellite repeat classes as percent of total satellites in human PDAC (Black, n = 15) and normal human tissues (White, n = 12) sequenced. Satellites are ordered from highest absolute difference in tumors to highest in normal tissue (left to right). Error bars represents standard error of the mean. Fold differential of top three cancer (left, black bars) and normal (right, white bars) tissue satellite classes shown (Bar graph, center).
FIG. 3C shows RNA in situ hybridization (RNA-ISH) of human satellite HSATII in preneoplastic PanIN (P) lesion with adjacent non-cancerous stroma tissue (N) (Top image) and fine needle aspirate biopsy of PDAC (T) and normal adjacent leukocytes (N).
FIG 4A shows the results of multiple linear correlation analysis of major satellite to other cellular transcripts among all mouse tumors and normal tissues as depicted by a heat map. X-axis is samples ordered by expression of major satellite and y-axis is genes ordered by linear correlation to major satellite expression. Light grey (High) and dark grey (Low) color is log2 (reads per million). Major satellite expression as percent genomic aligned reads (y-axis) rank ordered by satellite reads (x-axis) with expanded view of top genes with highest linearity (R ≥ 0.85) with satellite levels.
FIG. 4B is a dot graph showing the Median distance of transcriptional start sites of all genes to *LINE-1* elements ordered by linearity to satellite expression (Dark gray; highest linearity to the left) or by random (Light gray). Plotted by genes binned in 100s.
FIG. 4C is a dot graph showing Top genes with highest linearity (R > 0.85) defining satellite correlated genes or SCGs plotted by frequency against distance of transcriptional start site to *LINE-1* elements (Dark gray) compared to the expected frequency of these genes (Light gray).
FIG. 4D is a set of four photomicrographs showing the results of immunohistochemistry of mouse PDAC (KrasG12D, Tp53 lox/+) for the neuroendocrine marker chromogranin A. Tumors are depicted as a function of increasing chromogranin A staining (dark grey), with the relative level of major satellite expression noted for each tumor at the bottom of each image (percentage of all transcripts).
FIG. 5 is a photomicrograph showing the results of RNA-ISH using single plex ViewRNA chromogenic assay on human primary PDAC FFPE sample. Shown is HSP90BB positive (Red in original; dark areas around duct, some of which are indicated with arrows) epithelial ductal carcinoma cells (Duct indicated) overlaid on a bright field image of the tissue.

### DETAILED DESCRIPTION

The present invention is based, at least in part, on the identification of a massive generation of satellite RNAs in human and mouse cancers, and a number of satellite correlated genes. Thus the present methods are useful in the early detection of cancer, and can be used to predict clinical outcomes.

### Diagnosing Cancer Using Satellite Correlated Genes as Biomarkers

The methods described herein can be used to diagnose the presence of, and monitor the efficacy of a treatment for, cancer, e.g., solid tumors of epithelial origin, e.g., pancreatic, lung, breast, prostate, renal, ovarian or colon cancer, in a subject.

As used herein, the term "hyperproliferative" refer to cells having the capacity for autonomous growth, i.e., an abnormal state or condition characterized by rapidly proliferating cell growth. Hyperproliferative disease states may be categorized as pathologic, i.e., characterizing or constituting a disease state, or may be categorized as non-pathologic, i.e., a deviation from normal but not associated with a disease state. The term is meant to include all types of cancerous growths or oncogenic processes, metastatic tissues or malignantly transformed cells, tissues, or organs, irrespective of histopathologic type or stage of invasiveness. A "tumor" is an abnormal growth of hyperproliferative cells. "Cancer" refers to pathologic disease states, e.g., characterized by malignant tumor growth.

As demonstrated herein, the presence of cancer, e.g., solid tumors of epithelial origin, e.g., as defined by the ICD-O (International Classification of Diseases - Oncology) code (revision 3), section (8010-8790), e.g., early stage cancer, is associated with the presence of a massive levels of satellite due to increase in transcription and processing of satellite repeats in pancreatic cancer cells, and of increased levels of SCG expression in circulating tumor cells. Thus the methods can include the detection of expression levels of satellite repeats in a sample comprising cells known or suspected of being tumor cells, e.g., cells from solid tumors of epithelial origin, e.g., pancreatic, lung, breast, prostate, renal, ovarian or colon cancer cells. Alternatively or in addition, the methods can include the detection of increased levels of SCG in a sample, e.g., a sample known or suspected of including tumor cells, e.g., circulating tumor cells (CTCs), e.g., using a microfluidic device as described herein.

Cancers of epithelial origin can include pancreatic cancer (e.g., pancreatic adenocarcinoma or intraductal papillary mucinous carcinoma (IPMN, pancreatic mass)), lung cancer (e.g., non-small cell lung cancer), prostate cancer, breast cancer, renal cancer, ovarian cancer, or colon cancer. For example, the present methods can be used to distinguish between benign IPMN, for which surveillance is the standard treatment, and malignant IPMN, which require resection, a procedure associated with significant morbidity and a small but significant possibility of death. In some embodiments, in a subject diagnosed with IPMN, the methods described herein can be used for surveillance/monitoring of the subject, e.g., the methods can be repeated at selected intervals (e.g., every 3, 6, 12, or 24 months) to determine whether a benign IPMN has become a malignant IPMN warranting surgical intervention. In addition, the methods described herein can be used to distinguish bronchioloalveolar carcinomas from reactive processes (e.g., postpneumonic reactive processes) in samples from subjects suspected of having non-small cell lung cancer. In a sample from a subject who is suspected of having breast cancer, the methods described herein can be used to distinguish ductal hyperplasia from atypical ductal hyperplasia and ductal carcinoma in situ (DCIS). The two latter categories receive resection/radiation; the former does not require intervention. In subjects suspected of having prostate cancer, the methods described herein can be used to distinguish between atypical small acinar proliferation and malignant cancer. In subjects suspected of having bladder cancer, the methods described herein can be used to detect, e.g., transitional cell carcinoma (TCC), e.g., in urine specimens. In subjects diagnosed with Barrett's Esophagus (Sharma, N Engl J Med. 2009, 24; 361(26):2548-56. Erratum in: N Engl J Med. 2010 Apr 15;362(15):1450), the methods described herein can be used for distinguishing dysplasia in Barrett's esophagus from a reactive process. The clinical implications are significant, as a diagnosis of dysplasia demands a therapeutic intervention. Other instances include, but are not limited to, diagnosis of well differentiated hepatocellular carcinoma, ampullary and bile duct carcinoma, glioma vs. reactive gliosis, melanoma vs. dermal nevus and low grade sarcoma. Other embodiments include, but are not limited to, diagnosis of pancreatic endocrine tumors.

Therefore, described herein are methods for diagnosing cancer, e.g., tumors of epithelial origin, e.g., pancreatic, lung, breast, prostate, renal, ovarian or colon cancer, in a subject. In some instances, the methods include obtaining a sample from a subject, and evaluating the presence and/or level of SCG and/or satellites in the sample, and comparing the presence and/or level with one or more references, e.g., a control reference that represents a normal level of SCG or satellites, e.g., a level in an unaffected subject or a normal cell from the same subject, and/or a disease reference that represents a level of SCG or satellites associated with cancer, e.g., a level in a subject having pancreatic, lung, breast, prostate, renal, ovarian or colon cancer.

The present methods can also be used to determine the stage of a cancer, e.g., whether a sample includes cells that are from a precancerous lesion, an early stage tumor, or an advanced tumor. For example, the present methods can be used to determine whether a subject has a precancerous pancreatic, breast, or prostate lesion. Where the markers used are SCG transcript or encoded proteins, increasing levels are correlated with advancing stage.

### Samples

In some embodiments of the present methods, the sample is or includes blood, serum, and/or plasma, or a portion or subfraction thereof, e.g., free RNA in serum or RNA within exosomes in blood. In some embodiments, the sample comprises (or is suspected of comprising) CTCs. In some embodiments, the sample is or includes urine or a portion or subfraction thereof. In some embodiments, the sample includes known or suspected tumor cells, e.g., is a biopsy sample, e.g., a fine needle aspirate (FNA), endoscopic biopsy, or core needle biopsy; in some embodiments the sample comprises cells from the pancreatic, lung, breast, prostate, renal, ovarian or colon of the subject. In some embodiments, the sample comprises lung cells obtained from a sputum sample or from the lung of the subject by brushing, washing, bronchoscopic biopsy, transbronchial biopsy, or FNA, e.g., bronchoscopic, fluoroscopic, or CT-guided FNA (such methods can also be used to obtain samples from other tissues as well). In some embodiments, the sample is frozen, fixed and/or permeabilized, e.g., is an formalin-fixed paraffin-embedded (FFPE) sample.

### Methods of Detection

Any methods known in the art can be used to detect and/or quantify levels of a biomarker as described herein. For example, the level of an SCG mRNA (transcript) can be evaluated using methods known in the art, e.g., Northern blot, RNA in situ hybridization (RNA-ISH), RNA expression assays, e.g., microarray analysis, RT-PCR, RNA sequencing (e.g., using random primers or oligoT primers), deep sequencing, cloning, Northern blot, and amplifying the transcript, e.g., using quantitative real time polymerase chain reaction (qRT-PCR). Analytical techniques to determine RNA expression are known. See, e.g., Sambrook et al., Molecular Cloning: A Laboratory Manual, 3rd Ed., Cold Spring Harbor Press, Cold Spring Harbor, NY (2001).

In some embodiments, where the SCG is a coding transcript (see Table 6), the level of an SCG-encoded protein is detected. The presence and/or level of a protein can be evaluated using methods known in the art, e.g., using quantitative immunoassay methods such as enzyme linked immunosorbent assays (ELISAs), immunoprecipitations, immunofluorescence, immunohistochemistry, enzyme immunoassay (EIA), radioimmunoassay (RIA), and Western blot analysis.

In some embodiments, the methods include contacting an agent that selectively binds to a biomarker, e.g., to an SCG transcript/mRNA or protein (such as an oligonucleotide probe, an antibody or antigen-binding portion thereof) with a sample, to evaluate the level of the biomarker in the sample. In some embodiments, the agent bears a detectable label. The term "labeled," with regard to an agent encompasses direct labeling of the agent by coupling (i.e., physically linking) a detectable substance to the agent, as well as indirect labeling of the agent by reactivity with a detectable substance. Examples of detectable substances are known in the art and include chemiluminescent, fluorescent, radioactive, or colorimetric labels. For example, detectable substances can include various enzymes, prosthetic groups, fluorescent materials, luminescent materials, bioluminescent materials, and radioactive materials. Examples of suitable enzymes include horseradish peroxidase, alkaline phosphatase, beta-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride, quantum dots, or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H. In general, where a protein is to be detected, antibodies can be used. Antibodies can be polyclonal, or more preferably, monoclonal. An intact antibody, or an antigen-binding fragment thereof (e.g., Fab or F(ab')₂) can be used.

In some embodiments, high throughput methods, e.g., protein or gene chips as are known in the art (see, e.g., Ch. 12, "Genomics," in Griffiths et al., Eds. Modern genetic Analysis, 1999,W. H. Freeman and Company; Ekins and Chu, Trends in Biotechnology, 1999;17:217-218; MacBeath and Schreiber, Science 2000, 289(5485):1760-1763; Simpson, Proteins and Proteomics: A Laboratory Manual, Cold Spring Harbor Laboratory Press; 2002; Hardiman, Microarrays Methods and Applications: Nuts & Bolts, DNA Press, 2003), can be used to detect the presence and/or level of satellites or SCG.

In some embodiments, the methods include using a modified RNA *in situ* hybridization technique using a branched-chain DNA assay to directly detect and evaluate the level of biomarker mRNA in the sample (see, e.g., Luo et al., US Pat. No. 7,803,541B2, 2010; Canales et al., Nature Biotechnology 24(9):1115-1122 (2006); Nguyen et al., Single Molecule in situ Detection and Direct Quantiication of miRNA in Cells and FFPE Tissues, poster available at panomics.com/index.php?id=product_87). A kit for performing this assay is commercially-available from Affymetrix (ViewRNA).

### Detection of SCG Transcripts in CTCs

In some embodiments, microfluidic (e.g., "lab-on-a-chip") devices can be used in the present methods. Such devices have been successfully used for microfluidic flow cytometry, continuous size-based separation, and chromatographic separation. In general, methods in which expression of SCG transcripts is detected in circulating tumor cells (CTCs) can be used for the early detection of cancer, e.g., early detection of tumors of epithelial origin, e.g., pancreatic, lung, breast, prostate, renal, ovarian or colon cancer.

The devices can be used for separating CTCs from a mixture of cells, or preparing an enriched population of CTCs. In particular, such devices can be used for the isolation of CTCs from complex mixtures such as whole blood.

A variety of approaches can be used to separate CTCs from a heterogeneous sample. For example, a device can include an array of multiple posts arranged in a hexagonal packing pattern in a microfluidic channel upstream of a block barrier. The posts and the block barrier can be functionalized with different binding moieties. For example, the posts can be functionalized with anti-EPCAM antibody to capture circulating tumor cells (CTCs); see, e.g., Nagrath et al., Nature 450:1235-1239 (2007), optionally with downstream block barriers functionalized with to capture SCG nucleic acids or proteins, or satellites. See, e.g., (13-15) and the applications and references listed herein.

Processes for enriching specific particles from a sample are generally based on sequential processing steps, each of which reduces the number of undesired cells/particles in the mixture, but one processing step may suffice in some embodiments. Devices for carrying out various processing steps can be separate or integrated into one microfluidic system. The devices include devices for cell/particle binding, devices for cell lysis, devices for arraying cells, and devices for particle separation, e.g., based on size, shape, and/or deformability or other criteria. In certain embodiments, processing steps are used to reduce the number of cells prior to introducing them into the device or system. In some embodiments, the devices retain at least 75%, e.g., 80%, 90%, 95%, 98%, or 99% of the desired cells compared to the initial sample mixture, while enriching the population of desired cells by a factor of at least 100, e.g., by 1000, 10,000, 100,000, or even 1,000,000 relative to one or more non-desired cell types.

Some devices for the separation of particles rely on size-based separation with or without simultaneous cell binding. Some size-based separation devices include one or more arrays of obstacles that cause lateral displacement of CTCs and other components of fluids, thereby offering mechanisms of enriching or otherwise processing such components. The array(s) of obstacles for separating particles according to size typically define a network of gaps, wherein a fluid passing through a gap is divided unequally into subsequent gaps. Both sieve and array sized-based separation devices can incorporate selectively permeable obstacles as described above with respect to cell-binding devices.

Devices including an array of obstacles that form a network of gaps can include, for example, a staggered two-dimensional array of obstacles, e.g., such that each successive row is offset by less than half of the period of the previous row. The obstacles can also be arranged in different patterns. Examples of possible obstacle shapes and patterns are discussed in more detail in WO 2004/029221.

In some embodiments, the device can provide separation and/or enrichment of CTCs using array-based size separation methods, e.g., as described in U.S. Pat. Pub. No. 2007/0026413. In general, the devices include one or more arrays of selectively permeable obstacles that cause lateral displacement of large particles such as CTCs and other components suspended in fluid samples, thereby offering mechanisms of enriching or otherwise processing such components, while also offering the possibility of selectively binding other, smaller particles that can penetrate into the voids in the dense matrices of nanotubes that make up the obstacles. Devices that employ such selectively permeable obstacles for size, shape, or deformability based enrichment of particles, including filters, sieves, and enrichment or separation devices, are described in International Publication Nos. 2004/029221 and 2004/113877, Huang et al. Science 304:987-990 (2004), U.S. Publication No. 2004/0144651, U.S. Pat. Nos. 5,837,115 and 6,692,952, and U.S. Publication No. 2007/0196820; devices useful for affinity capture, e.g., those described in International Publication No. 2004/029221 and U.S. Publication. No. 2005/0266433; devices useful for preferential lysis of cells in a sample, e.g., those described in International Publication No. 2004/029221, U.S. Pat. No. 5,641,628; devices useful for arraying cells, e.g., those described in International Publication No. 2004/029221, U.S. Pat. No. 6,692,952, and U.S. Publication Nos. 2004/0166555 and 2006/0128006; and devices useful for fluid delivery, e.g., those described in U.S. Publication Nos. 2005/0282293 and 2006/0121624. Two or more devices can be combined in series, e.g., as described in International Publication No. WO 2004/029221.

In some embodiments, a device can contain obstacles that include binding moieties, e.g., monoclonal anti-EpCAM antibodies or fragments thereof, that selectively bind to particular cell types, e.g., cells of epithelial origin, e.g., tumor cells. All of the obstacles of the device can include these binding moieties; alternatively, only a subset of the obstacles include them. Devices can also include additional modules, e.g., a cell counting module or a detection module, which are in fluid communication with the microfluidic channel device. For example, the detection module can be configured to visualize an output sample of the device.

In one example, a detection module can be in fluid communication with a separation or enrichment device. The detection module can operate using any method of detection disclosed herein, or other methods known in the art. For example, the detection module includes a microscope, a cell counter, a magnet, a biocavity laser (see, e.g., Gourley et al., J. Phys. D: Appl. Phys., 36: R228-R239 (2003)), a mass spectrometer, a PCR device, an RT-PCR device, a microarray, a device for performing RNA *in situ* hybridization, or a hyperspectral imaging system (see, e.g., Vo-Dinh et al., IEEE Eng. Med. Biol. Mag., 23:40-49 (2004)). In some embodiments, a computer terminal can be connected to the detection module. For instance, the detection module can detect a label that selectively binds to cells, proteins, or nucleic acids of interest, e.g., SCG transcripts or encoded proteins.

In some embodiments, the microfluidic system includes (i) a device for separation or enrichment of CTCs; (ii) a device for lysis of the enriched CTCs; and (iii) a device for detection of SCG transcripts or encoded proteins.

In some embodiments, a population of CTCs prepared using a microfluidic device as described herein is used for analysis of expression of SCG transcripts or proteins using known molecular biological techniques, e.g., as described above and in Sambrook, Molecular Cloning: A Laboratory Manual, Third Edition (Cold Spring Harbor Laboratory Press; 3rd edition (January 15, 2001)); and Short Protocols in Molecular Biology, Ausubel et al., eds. (Current Protocols; 52 edition (November 5, 2002)).

In general, devices for detection and/or quantification of expression of SCG transcripts or encoded proteins in an enriched population of CTCs are described herein and can be used for the early detection of cancer, e.g., tumors of epithelial origin, e.g., early detection of pancreatic, lung, breast, prostate, renal, ovarian or colon cancer.

### Methods of Monitoring Disease Progress or Treatment Efficacy

Once it has been determined that a person has cancer, or has an increased risk of developing cancer, then a treatment, e.g., as known in the art, can be administered. The efficacy of the treatment can be monitored using the methods described herein; an additional sample can be evaluated after (or during) treatment, e.g., after one or more doses of the treatment are administered, and a decrease in the level of expression of SCG transcripts or encoded protein, or in the number of SCG transcript or protein-expressing cells in a sample, would indicate that the treatment was effective, while no change or an increase in the level of SCG transcript or protein-expressing cells would indicate that the treatment was not effective. The methods can be repeated multiple times during the course of treatment, and/or after the treatment has been concluded, e.g., to monitor potential recurrence of disease.

In some embodiments, e.g., for subjects who have been diagnosed with a benign condition that could lead to cancer, subjects who have been successfully treated for a cancer, or subjects who have an increased risk of cancer, e.g., due to a genetic predisposition or environmental exposure to cancer-causing agents, the methods can be repeated at selected intervals, e.g., at 3, 6, 12, or 24 month intervals, to monitor the disease in the subject for early detection of progression to malignancy or development of cancer in the subject.

### EXAMPLES

The invention is further described in the following examples, which do not limit the scope of the invention described in the claims.

### Example 1. Major Satellite levels are massively elevated in tumor tissues compared to cell lines and normal tissue

The next generation digital gene expression (DGE) application from Helicos BioSciences (D. Lipson et al., Nat Biotechnol 27, 652 (Jul, 2009)) was utilized to compare expression of tumor markers in primary cancers and their derived metastatic precursors. We first determined DGE profiles of primary mouse pancreatic ductal adenocarcinoma (PDAC) generated through tissue-targeted expression of activated *Kras* and loss of *Tp53* (*Kras^{G12D}*, *Tp53* ^{*lox*/+}) (N. Bardeesy et al., Proc Natl Acad Sci U S A 103, 5947 (Apr 11, 2006)). These tumors are histopathological and genetic mimics of human PDAC, which exhibits virtually universal mutant *KRAS* (>90% of cases) and loss of *TP53* (50-60%).

Mice with pancreatic cancer of different genotypes were bred as previously described in the Bardeesy laboratory (Bardeesy et al., Proc Natl Acad Sci USA 103, 5947 (2006)). Normal wild type mice were purchased from Jackson laboratories. Animals were euthanized as per animal protocol guidelines. Pancreatic tumors and normal tissue were extracted sterilely and then flash frozen with liquid nitrogen. Tissues were stored at -80° C. Cell lines were generated fresh for animals AH367 and AH368 as previously described (Aguirre et al., Genes Dev 17, 3112 (2003)) and established cell lines were cultured in RPMI-1640 + 10% FBS + 1% Pen/Strep (Gibco/Invitrogen). Additional mouse tumors from colon and lung were generously provided by Kevin Haigis (Massachusetts General Hospital) and Kwok-Kin Wong (Dana Farber Cancer Institute).

Fresh frozen tissue was pulverized with a sterile pestle in a microfuge tube on dry ice. Cell lines were cultured and fresh frozen in liquid nitrogen prior to nucleic acid extraction. RNA and DNA from cell lines and fresh frozen tumor and normal tissues were all processed in the same manner. RNA was extracted using the TRIzol® Reagent (Invitrogen) per manufacturer's specifications. DNA from tissue and cell lines was extracted using the QIAamp Mini Kit (QIAGEN) per manufacturer's protocol.

Purified RNA was subjected to Digital Gene Expression (DGE) sample prepping and analysis on the HeliScope™ Single Molecule Sequencer from Helicos BioSciences. This method has been previously described (Lipson et al., Nat Biotechnol 27, 652 (2009)). Briefly, Single stranded cDNA was reverse transcribed from RNA with a dTU25V primer and the Superscript III cDNA synthesis kit (Invitrogen). RNA was digested and single stranded cDNA was purified using a solid phase reversible immobilization (SPRI) technique with Agencourt® AMPure® magnetic beads. Single stranded cDNA was denatured and then a poly-A tail was added to the 3' end using terminal transferase (New England Biolabs).

Purified DNA was subjected to the DNA sequencing sample prepping protocol from Helicos that has been previously described (Pushkarev, N. F. Neff, S. R. Quake, Nat Biotech 27, 847 (2009)). Briefly, genomic DNA was sheared with a Covaris S2 acoustic sonicator producing fragments averaging 200 bps and ranging from 100-500 bps. Sheared DNA was then cleaned with SPRI. DNA was then denatured and a poly-A tail was added to the 3' end using terminal transferase.

Tailed cDNA or DNA were then hybridized to the sequencing flow cell followed by "Fill and Lock" and single molecule sequencing. Gene expression sequence reads were then aligned to the known human or mouse transcriptome libraries using the DGE program. Genomic DNA sequence reads were aligned to the mouse genome and counted to determine copy number of the major mouse satellite (CNV).

The first mouse pancreatic tumor analyzed, AH284, was remarkable in that DGE sequences displayed a 48-52% discrepancy with the annotated mouse transcriptome, compared with a 3-4% difference for normal liver transcripts from the same mouse. Nearly all the discrepant sequences mapped to the pericentric (major) mouse satellite repeat. The satellite transcript accounts for ∼49% (495,421 tpm) of all cellular transcripts in the tumor, compared with 0.02-0.4% (196-4,115 tpm) in normal pancreas or liver (Table 1).

**Table 1**

| Total genomic aligned reads with breakdown of major satellite and transcriptome reads. Percentage of total genomic aligned reads in parentheses | | | |
|---|---|---|---|
| | **Total Reads** | **Major Satellite Reads** | **Transcriptome** |
| Pancreatic Tumor | 18,063,363 | 8,460,135 | 1,726,768 |
| | | (47%) | (10%) |
| Normal Liver | 2,270,669 | 8,973 | 1,718,489 |
| | | (0.4%) | (75%) |
| Normal Pancreas | 492,301 | 2,026 | 63,160 |
| | | (0.4%) | (13%) |

Satellite sequence reads were found in both sense and anti-sense directions and are absent from poly-A purified RNA. Tumor AH284 therefore contained massive amounts of a non-polyadenylated dsRNA element, quantitatively determined as >100-fold increased over that present in normal tissue from the same animal. By way of comparison, the levels of satellite transcripts in tumor tissues were about 8,000-fold higher than the abundant mRNA *Gapdh.* A second independent pancreatic tumor nodule from the same mouse showed a lower, albeit still greatly elevated, level of satellite transcript (4.5% of total cellular transcripts).

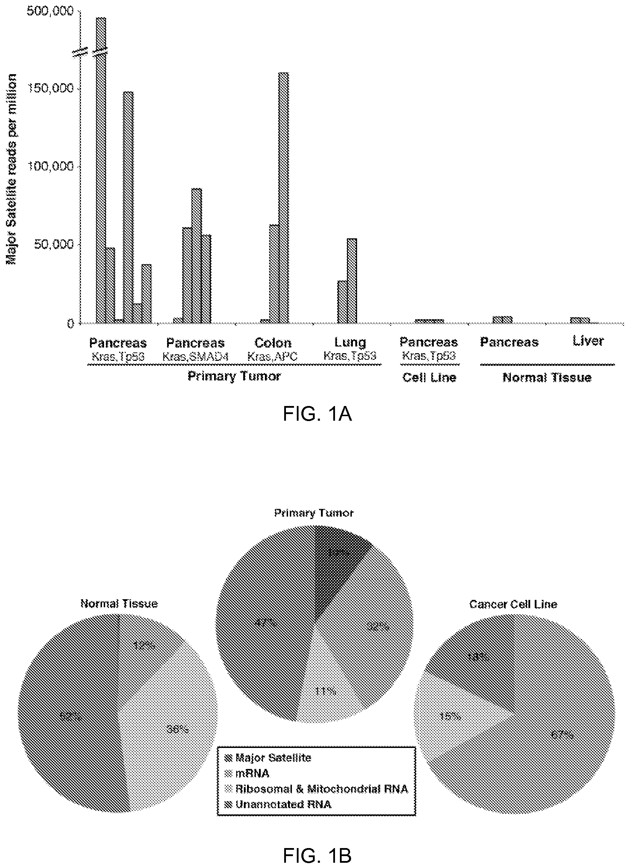

Analysis of 4 additional pancreatic tumors from (*Kras^{G12D}*, *Tp53*^{*lox*/+}) mice and 4 mice with an alternative pancreatic tumorigenic genotype *(Kras ^{G12D}*, *SMAD4*^{*lox*/*lox*}) revealed increased satellite expression in 6/8 additional tumors (range 1-15% of all cellular transcripts). In 2/3 mouse colon cancer tumors (*Kras^{G12D}*, *APC* ^{*lox*/+}) and 2/2 lung cancers (*Kras ^{G12D}*, *Tp53* ^{*lox*/*lox*}), satellite expression level ranged from 2-16% of all cellular transcripts. In total, 12/15 (80%) independent mouse tumors had greatly increased levels of satellite expression, compared to normal mouse tissues (Fig. 1A, Table 2).

**Table 2**

| Total genomic reads and percentage of reads aligning to transcriptome and major satellite among multiple mouse tumors, cell lines, and normal tissues. | | | | | |
|---|---|---|---|---|---|
| **Mouse ID** | **Tissue Type** | **Genotype** | **Total Genomic Reads** | **% Transcriptome Reads** | **% Major Satellite Reads** |
| AH284 Rep 1 | Pancreatic Cancer | *Kras^{G12D}, Tp53*^{*lox*/*+*} | 18,063,363 | 9.56% | 46.84% |
| AH284 Rep 2 | Pancreatic Cancer | *Kras^{G12D}, Tp53*^{*lox*/*+*} | 16,948,693 | 10.15% | 49.54% |
| AH284 - 2* | Pancreatic Cancer | *Kras^{G12D}, Tp53*^{*lox*/*+*} | 1,613,592 | 48.67% | 4.78% |
| AH287 | Pancreatic Cancer | *Kras^{G12D}, Tp53*^{*lox*/*+*} | 2,227,850 | 54.70% | 0.07% |
| AH288 | Pancreatic Cancer | *Kras^{G12D}, Tp53*^{*lox*/*+*} | 6,780,821 | 26.57% | 14.79% |
| AH291 | Pancreatic Cancer | *Kras^{G12D}, Tp53*^{*lox*/*+*} | 1,388,906 | 43.12% | 1.22% |
| AH294 | Pancreatic Cancer | *Kras^{G12D}, Tp53*^{*lox*/*+*} | 969,896 | 37.20% | 3.73% |
| AH323 | Pancreatic Cancer | *Kras^{G12D}, SMAD4*^{*lox*/*lox*} | 1,887,663 | 72.73% | 0.29% |
| AH346 | Pancreatic Cancer | *Kras^{G12D}, SMAD4*^{*lox*/*lox*} | 1,291,648 | 32.92% | 6.07% |
| AH347 | Pancreatic Cancer | *Kras^{G12D}, SMAD4*^{*lox*/*lox*} | 1,634,314 | 38.94% | 8.59% |
| AH348 | Pancreatic Cancer | *Kras^{G12D}, SMAD4*^{*lox*/*lox*} | 2,030,197 | 45.84% | 5.61% |
| Colon 1 | Colon Cancer - 1 | *Kras^{G12D}, APC*^{*lox*/*lox*} | 2,954,930 | 77.49% | 0.07% |
| Colon 1 | Colon Cancer - 2 | *Kras^{G12D}, APC*^{*lox*/*lox*} | 985,510 | 53.13% | 6.27% |
| Colon 1 | Colon Cancer - 3 | *Kras^{G12D}, APC*^{*lox*/*lox*} | 1,017,319 | 30.71% | 16.02% |
| KN2128 | Lung Cancer | *Kras^{G12D}, Tp53*^{*lox*/*lox*} | 2,233,183 | 60.78% | 2.66% |
| KN2199 | Lung Cancer | *Kras^{G12D}, Tp53*^{*lox*/*lox*} | 1,653,948 | 43.21% | 5.37% |
| AH323 | PDAC Cell Line | *Kras^{G12D}, SMAD4*^{*lox*/*lox*} | 1,958,108 | 83.13% | 0.02% |
| AH324 | PDAC Cell Line | *Kras^{G12D}, Tp53*^{*lox*/*+*} | 3,301,108 | 86.32% | 0.04% |
| NB490 | PDAC Cell Line | *Kras^{G12D}, Tp53*^{*lox*/*lox*} | 15,378,802 | 76.85% | 0.03% |
| AH284 Rep 1 | Matched Normal Liver | *Kras^{G12D}, Tp53*^{*lox*/*+*} | 2,270,669 | 75.68% | 0.40% |
| AH284 Rep 2 | Matched Normal Liver | *Kras^{G12D}, Tp53*^{*lox*/*+*} | 1,627,749 | 56.59% | 0.34% |
| AH284 - 2* | Matched Normal Liver | *Kras^{G12D}, Tp53*^{*lox*/*+*} | 644,316 | 41.10% | 0.31% |
| Colon 1 | Matched Normal Liver | *KraS^{G12D}, APC*^{*lo*/*lox*} | 1,536,346 | 86.53% | 0.02% |
| Normal 1 | Normal Pancreas | WT | 247,582 | 14.49% | 0.41% |
| Normal 2 | Normal Pancreas | WT | 244,719 | 11.15% | 0.41% |

| | | | | | |
|---|---|---|---|---|---|
| * AH284-2 was RNA extraction from a different part of the pancreatic tumor and liver | | | | | |

Of note, the composite distribution of all RNA reads among coding, ribosomal and other non-coding transcripts showed significant variation between primary tumors and normal tissues (Fig. 1A), suggesting that the global cellular transcriptional machinery is affected by the massive expression of satellite transcripts in primary tumors. Immortalized cell lines established from 3 primary pancreatic tumors displayed minimal expression of satellite repeats, suggesting either negative selection pressure during in vitro proliferation or reestablishment of stable satellite silencing mechanisms under in vitro culture conditions (Fig. 1A). Of note in primary tumors overexpressing satellites, the composite distribution of all RNA reads among coding, ribosomal and other non-coding transcripts shows significant variation with that of normal tissues (Fig. 1B), suggesting that the cellular transcriptional machinery is affected by the massive expression of satellite transcripts in these tumors.

### Example 2. Major satellite transcripts are of various sizes depending on tissue type and expression levels are linked to genomic methylation and amplification

Northern blot analysis of mouse primary pancreatic tumors was carried out as follows. Northern Blot was performed using the NorthernMax-Gly Kit (Ambion). Total RNA (10 ug) was mixed with equal volume of Glyoxal Load Dye (Ambion) and incubated at 50°C for 30 min. After electrophoresis in a 1% agarose gel, RNA was transferred onto BrightStar-Plus membranes (Ambion) and crosslinked with ultraviolet light. The membrane was prehybridized in ULTRAhyb buffer (Ambion) at 68°C for 30 min. The mouse RNA probe (1100 bp) was prepared using the MAXIscript Kit (Ambion) and was nonisotopically labeled using the BrightStar Psoralen-Biotin Kit (Ambion) according to the manufacturer's instructions. Using 0.1 nM probe, the membrane was hybridized in ULTRAhyb buffer (Ambion) at 68°C for 2 hours. The membrane was washed with a Low Stringency wash at room temperature for 10 min, followed by two High Stringency washes at 68°C for 15 min. For nonisotopic chemiluminescent detection, the BrightStar BioDetect Kit was used according to the manufacturer's instructions.

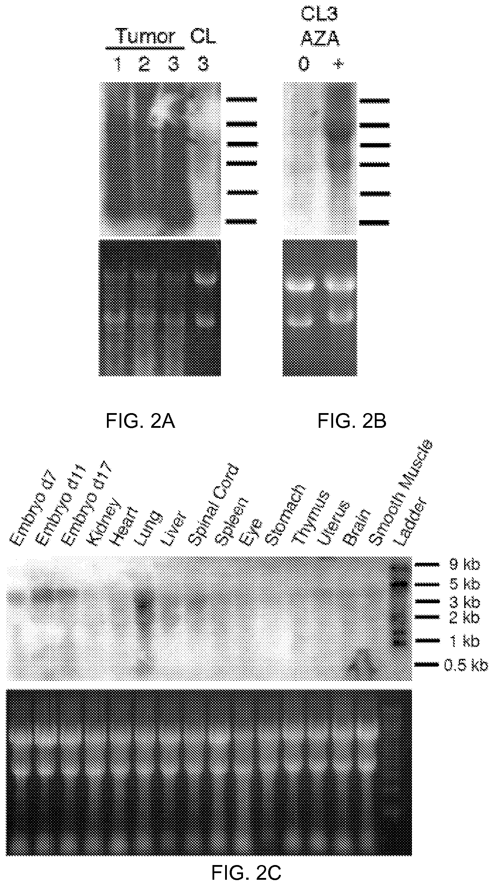

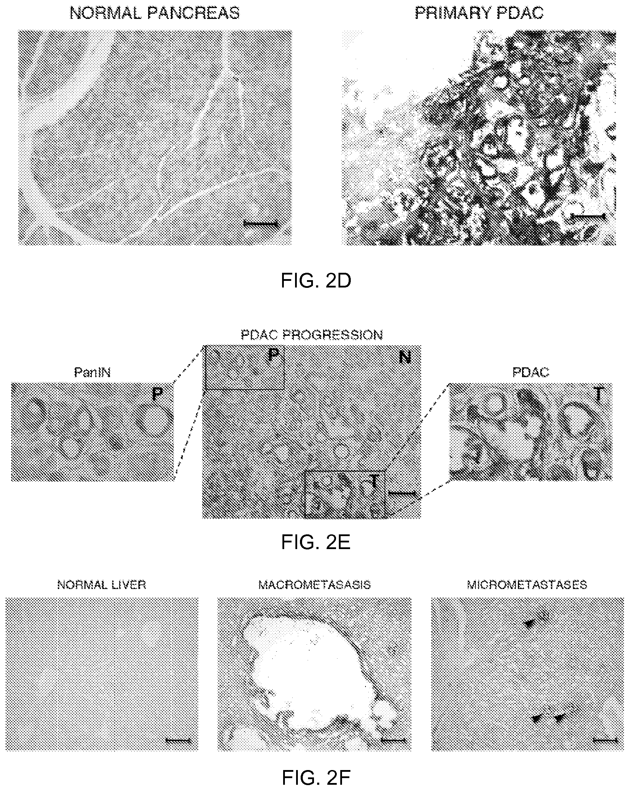

The results demonstrated that the major satellite-derived transcripts range from 100 bp to 2.5 kb (Fig. 2A), consistent with the predicted cleavage of a large primary transcript comprised of multiple tandem repeats by *Dicer1* (C. Kanellopoulou et al., Genes Dev 19, 489 (Feb 15, 2005); T. Fukagawa et al., Nat Cell Biol 6, 784 (Aug, 2004); H. Bouzinba-Segard, A. Guais, C. Francastel, Proc Natl Acad Sci U S A 103, 8709 (Jun 6, 2006)), whose expression is 2.6-fold higher (p = 0.0006, t-test) in mouse pancreatic tumors with satellite expression above the median. An established pancreatic cancer cell line derived from a primary tumor with high satellite expression has very little satellite expression confirming our sequencing results (T3 and CL3; Fig. 2A). Treatment of CL3 with 5-azacytidine leads to massive reexpression of satellite transcripts, supporting DNA methylation as a mechanism for stable satellite silencing in vitro (Fig. 2B). Most normal adult mouse tissues, with the exception of lung, show minimal expression of satellite repeats (Fig. 2B). However, expression of the uncleaved 5 kb satellite transcript is evident in embryonic tissues (Fig. 2C). Thus, the aberrant expression of satellite repeats in primary pancreatic tumors does not simply recapitulate an embryonic cell fate, but also reflects altered processing of the primary 5 kb satellite transcript. The single molecule sequencing platform was exceptionally sensitive for quantitation of small repetitive ncRNA fragments, each of which is scored as a unique read. High level expression of the mouse major satellite was evident in all cells within the primary tumor (Fig. 2D), as shown by RNA in situ hybridization (ISH). Remarkably, expression was already elevated in early preneoplastic lesions, pancreatic intraepithelial neoplasia (PanIN), and it increased further upon transition to full pancreatic adenocarcinoma (Fig. 2E). Clearly defined metastatic lesions to the liver ware strongly positive by RNA ISH, as were individual PDAC cells within the liver parenchyma that otherwise would not have been detected by histopathological analysis (Fig. 2F). Low level diffuse expression was evident in liver and lung, as shown by whole mount embryo analysis, but no normal adult or embryonic tissues demonstrated satellite expression comparable to that evident in tumor cells.

To determine whether genomic amplification of satellite repeats also contributes toward the exceptional abundance of these transcripts in mouse pancreatic tumors, the index AH284 tumor was analyzed using next generation DNA digital copy number variation (CNV) analysis as described above for genomic DNA sequencing.

The results, shown in Table 3, indicated that satellite DNA comprised 18.8% of all genome-aligned reads in this tumor, compared with 2.3% of genomic sequences in matched normal liver. The major satellite repeat has previously been estimated at approximately 3% of the normal mouse genome (J. H. Martens et al., EMBO J 24, 800 (Feb 23, 2005)). Thus, in this tumor with >100-fold increased expression of satellite repeats, approximately 8-fold gene amplification of the repeats may contribute to their abnormal expression.

**Table 3**

| CNV analysis of index pancreatic tumor and normal liver from mouse AH284. Major satellite reads as a percentage of all genomic aligned reads (last column) | | |
|---|---|---|
| | **Major Satellite Reads** | **Total Genomic Reads** |
| AH284 Liver | 183,327 | 7,995,538 |
| | (2.3%) | |
| AH284 PDAC | 2,283,436 | 12,124,201 |
| | (18.8%) | |

### Example 3. Overexpression of satellite transcripts in human pancreatic cancer and other epithelial cancers

To test whether human tumors also overexpress satellite ncRNAs, we extended the DGE analysis to specimens of human pancreatic cancer. Human pancreatic tumor tissues were obtained as excess discarded human material per IRB protocol from the Massachusetts General Hospital. Gross tumor was excised and fresh frozen in liquid nitrogen prior to nucleic acid extraction. Normal pancreas RNA was obtained from two commercial vendors, Clontech and Ambion. The samples were prepared and analyzed as described above in Example 1.

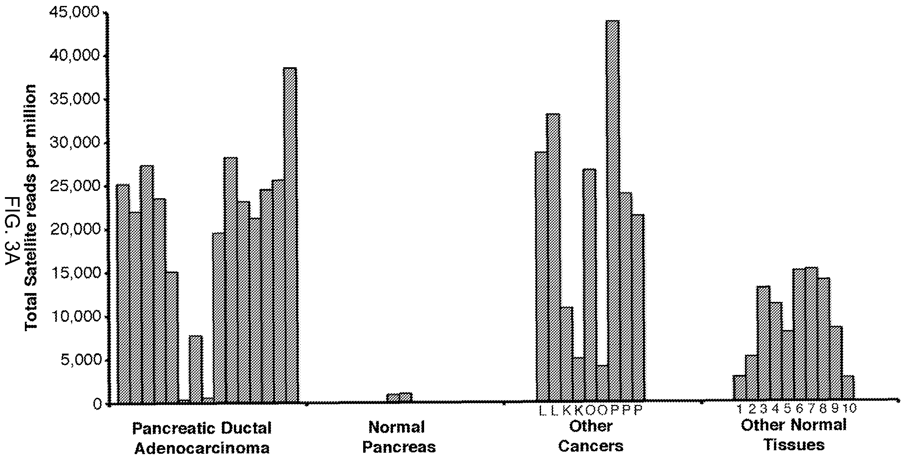

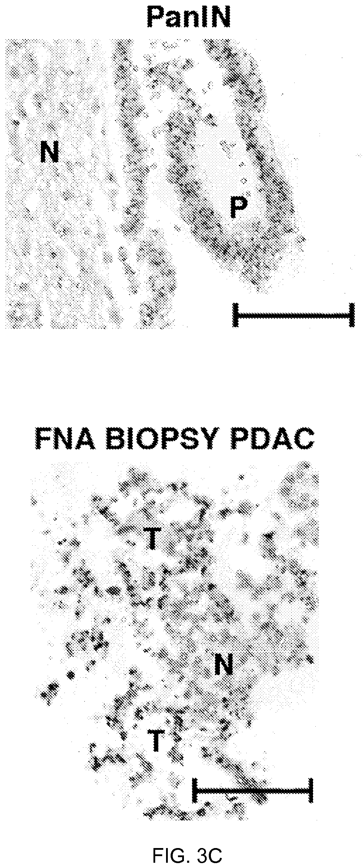

Analysis of 15 PDACs showed a median 21-fold increased expression of total satellite transcripts compared with normal pancreas. A cohort of non-small cell lung cancer, renal cell carcinoma, ovarian cancer, and prostate cancer also had significant levels of satellites and the HSATII satellite. Other normal human tissues, including fetal brain, brain, colon, fetal liver, liver, lung, kidney, placenta, prostate, and uterus have somewhat higher levels of total satellite expression (Table 4, Fig. 3A).

**Table 4**

| **SAMPLE ID** | **Genome** | **Total Satellite (tpm)** | **ALR (tpm)** | **HSATII (tpm)** |
|---|---|---|---|---|
| PDAC 1 | 4,472,810 | 25,209 | 14,688 | 3,589 |
| PDAC 2 | 1,668,281 | 22,001 | 12,653 | 3,295 |
| PDAC 3 | 5,211,399 | 27,366 | 15,921 | 5,057 |
| PDAC 4 | 1,649,041 | 23,556 | 13,428 | 3,167 |
| PDAC 5 | 239,483 | 15,095 | 8,259 | 509 |
| PDAC 6 | 1,520,470 | 374 | 195 | 14 |
| PDAC 7 | 1,449,321 | 7,738 | 4,400 | 750 |
| PDAC 8 | 1,950,197 | 574 | 316 | 9 |
| PDAC 9 | 3,853,773 | 19,572 | 12,563 | 1,731 |
| PDAC 10 | 2,748,850 | 28,225 | 18,767 | 2,489 |
| PDAC 11 | 2,848,599 | 23,163 | 14,634 | 2,589 |
| PDAC 12 | 3,723,326 | 21,243 | 12,940 | 2,122 |
| PDAC 13 | 1,834,743 | 24,549 | 15,342 | 3,150 |
| PDAC 14 | 2,481,332 | 25,650 | 18,016 | 2,564 |
| PDAC 15 | 1,752,081 | 38,514 | 25,899 | 5,210 |
| Normal Pancreas 1 | 1,196,372 | 908 | 284 | 0 |
| Normal Pancreas 2 | 975,676 | 1,043 | 303 | 0 |
| Lung Cancer 1 | 1,549,237 | 28,658 | 18,751 | 4,417 |
| Lung Cancer 2 | 13,829,845 | 33,030 | 26,143 | 2,555 |
| Kidney Cancer 1 | 2,104,859 | 10,814 | 6,505 | 1,501 |
| Kidney Cancer 2 | 4,753,409 | 5,025 | 2,739 | 625 |
| Ovarian Cancer 1 | 12,596,542 | 26,658 | 14,513 | 3,074 |
| Ovarian Cancer 2 | 7,290,000 | 4,089 | 2,058 | 403 |
| Prostate Cancer 1 | 3,376,849 | 43,730 | 22,244 | 9,793 |
| Prostate Cancer 2 | 12,052,244 | 23,947 | 14,201 | 3,209 |
| Prostate Cancer 3 | 3,631,148 | 21,411 | 12,390 | 2,804 |
| Normal Fetal Brain | 384,453 | 2,843 | 1,516 | 3 |
| Normal Brain | 371,161 | 5,184 | 2,573 | 3 |
| Normal Colon | 183,855 | 13,059 | 7,229 | 5 |
| Normal Fetal Liver | 147,977 | 11,218 | 5,879 | 7 |
| Normal Liver | 117,976 | 7,968 | 3,730 | 25 |
| Normal Lung | 208,089 | 15,027 | 7,857 | 5 |
| Normal Kidney | 144,173 | 15,218 | 8,094 | 7 |
| Normal Placenta | 207,929 | 13,990 | 7,815 | 0 |
| Normal Prostate | 263,406 | 8,409 | 2,228 | 19 |
| Normal Uterus | 477,480 | 2,702 | 1,395 | 2 |

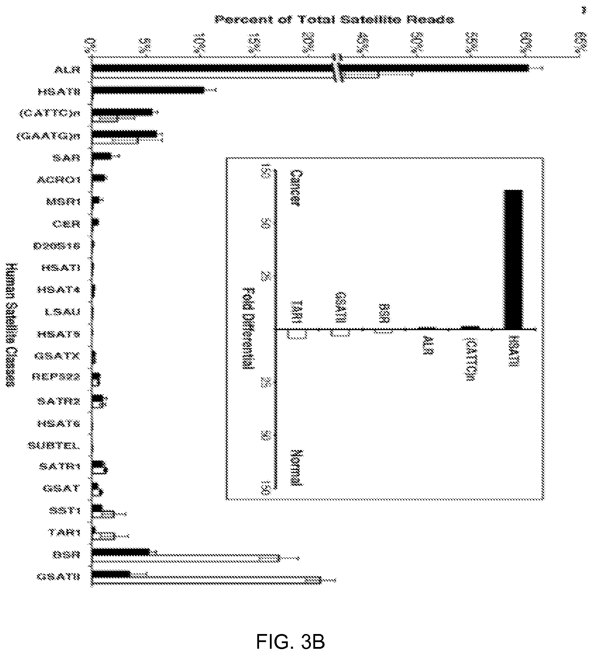

Subdivision of human satellite among the multiple classes revealed major differences between tumors and all normal tissues. While mouse satellite repeats are broadly subdivided into major and minor satellites, human satellites have been classified more extensively. Of all human satellites, the greatest expression fold differential is evident for the pericentromeric satellite HSATII (mean 2,416 tpm; 10.3% of satellite reads), which is undetectable in normal human pancreas (Fig. 3B). In contrast, normal tissues have much higher representation of GSATII, Beta satellite (BSR), and TAR1 classes (21.1%, 17.3%, and 2.1% of all satellite reads respectively), while these constitute a small minority of satellite reads in pancreatic cancers.

The most abundant class of normally expressed human satellites, alpha (ALR) (Okada et al., Cell 131, 1287 (Dec 28, 2007)) is expressed at 294 tpm in normal human pancreas, but comprises on average 12,535 tpm in human pancreatic adenocarcinomas (43-fold differential expression; 60.3% of satellite reads). Thus, while the overexpression of human ALR repeats is comparable to that of mouse major satellite repeats, it is the less abundant HSATII (49-fold above *GAPDH),* which shows exceptional specificity for human PDAC. The co-expression of *LINE-*1 with satellite transcripts in human pancreatic tumors is also striking, with a mean 16,089 tpm (range 358-38,419).

Beyond ALR repeats, the satellite expression profile of normal pancreas and PDAC are strikingly different; for instance normal pancreatic tissue has a much higher representation of GSATII, TAR1 and SST1 classes (26.4%, 10.6%, and 8.6% of all satellite reads), while these were a small minority of satellite reads in pancreatic cancers. In contrast, cancers express high levels of HSATII satellites (4,000 per 10⁶ transcripts; 15% of satellite reads), a subtype whose expression is undetectable in normal pancreas (Fig. 3B). Quantitative comparison of satellite transcription in mouse versus human pancreatic cancers shows that mouse major satellites are expressed a median 466-fold above the abundant *Gapdh* mRNA, while the human ALR and HSATII satellites are respectively expressed 180-fold and 47-fold above *GAPDH.*

### Example 4. Cellular transcripts with linear correlation to increasing satellite levels are enriched for stem cell and neural elements that is linked to histone demethylases and RNA processing enzymes

The generation of comprehensive DGE profiles for 25 different mouse tissues of different histologies and genetic backgrounds made it possible to correlate the expression of cellular transcripts with that of satellites across a broad quantitative range. To identify such co-regulated genes, all annotated transcripts quantified by DGE were subjected to linear regression analysis, and transcripts with the highest correlation coefficients to satellite expression were rank ordered.

All mouse sample reads were aligned to a custom made library for the mouse major satellite (sequence from UCSC genome browser). Human samples were aligned to a custom made reference library for all satellite repeats and LINE-1 variants generated from the Repbase library (Pushkarev et al., Nat Biotech 27, 847 (2009)). In addition, all samples were subjected to the DGE program for transcriptome analysis. Reads were normalized per 10⁶ genomic aligned reads for all samples.

For linear correlation of mouse major satellite to transcriptome, all tissues and cell lines were rank ordered according to level of major satellite. All annotated genes were then subjected to linear regression analysis across all tissues. Genes were then ordered according to the Pearson coefficient for linear regression and plotted by Matlab.

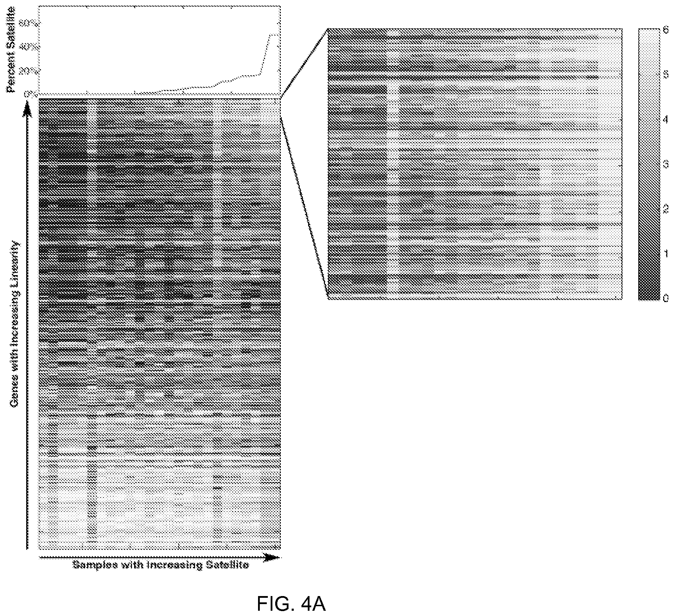

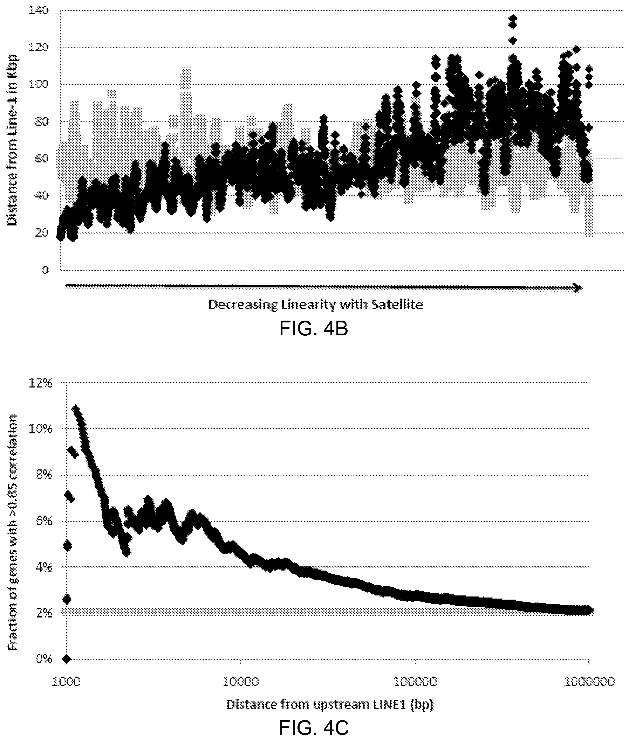

Analysis of a set of 297 genes with highest linear correlation (R > 0.85) revealed 190 annotated cellular mRNAs and a subset of transposable elements (Fig. 4A).

A subset of cellular mRNAs showed a very high degree of correlation with the levels of satellite repeat expression across diverse mouse tumors (referred to herein as "Satellite Correlated Genes (SCGs)"). Linearly correlated genes with R > 0.85 were mapped using the DAVID program (Dennis, Jr. et al., Genome Biol 4, P3 (2003); Huang et al., Nat Protoc 4, 44 (2009)). These genes were then analyzed with the Functional Annotation clustering program and the UP_TISSUE database to classify each of these mapped genes. Germ/Stem cell genes included genes expressed highly in testis, egg, trophoblast, and neural stem cells. Neural genes included genes expressed highly in brain, spinal cord, and specialized sensory neurons including olfactory, auditory, and visual perception. HOX and Zinc Finger proteins were classified using the INTERPRO database.

Analysis of 190 annotated transcripts using the DAVID gene ontology program identified 120 (63%) of these transcripts as being associated with neural cell fates and 50 (26%) linked with germ/stem cells pathways (Table 5).

**Table 5**

| | **Germ/Stem Cell** | **Neural** | **HOX Region** | **Zinc Finger Domain** |
|---|---|---|---|---|
| **TOTAL COUNTS** | 50 | 120 | 10 | 16 |
| **% Mapped (190)** | 26% | 63% | 5% | 8% |

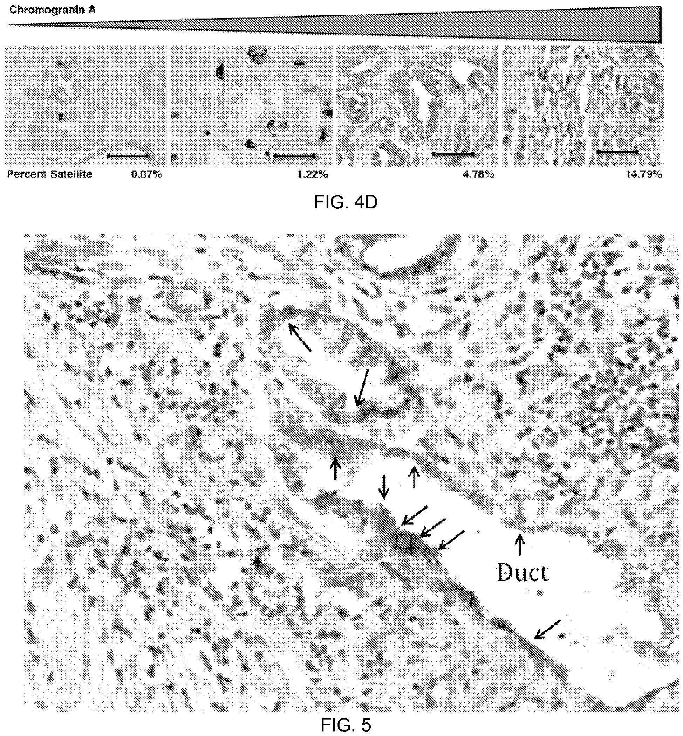

In addition, significant enrichment was evident for transcriptional regulators, including HOX related (9, 5%) and zinc finger proteins (16,8%). This gene set could not be matched to any known gene signature in the GSEA database (Subramanian et al., Proc Natl Acad Sci U S A 102, 15545 (Oct 25, 2005)), but the ontology analysis points towards a neuroendocrine phenotype. Neuroendocrine differentiation has been described in a variety of epithelial malignancies, including pancreatic cancer (Tezel et al., Cancer 89, 2230 (Dec 1, 2000)), and is best characterized in prostate cancer where it is correlated with more aggressive disease (Cindolo et al., Urol Int 79, 287 (2007)). A striking increase in the number of carcinoma cells staining for the characteristic neuroendocrine marker chromogranin A, as a function of higher satellite expression in mouse PDACs (Fig. 4D), was observed, supporting the link between globally altered expression of ncRNAs and a specific cellular differentiation program.

A parallel analysis in human pancreatic cancers and normal tissues using the ALR, the most abundant human satellite, yielded a total of 539 SCGs, Of these 206 could be mapped by the DAVID gene ontology program with a similar enrichment of germ/stem and neural cell fates (Table 6). Together, these observations suggest that, as in the mouse genetic model, tumor-associated derepression of satellite-derived repeats is highly correlated with increased expression of a subset of cellular mRNAs.

**Table 6**

| | **Germ/Stem Cell** | **Neural** | **Zinc Finger Domain** |
|---|---|---|---|
| **TOTAL COUNTS** | 101 | 63 | 35 |
| **% Mapped (206)** | 49.0% | 30.6% | 17.0% |

The list of SCGs with utility as biomarkers was further refined by taking human SCGs with a minimum 20 fold differential between cancer and normal tissue and with a minimum expression of 500 reads per million. The results are shown in

**Table 7.**

| Table 7. Satellite Correlated Genes | | | | | | |
|---|---|---|---|---|---|---|
| Human Gene Name | Reads per million | | RATIO | Name | GenBank Accession No. | |
| | | | | | mRNA | Protein |
| | Cancer | Normal | | | | |
| HSP90BB | 5589.1 | 123.6 | 45.2 | heat shock protein 90kDa alpha (cytosolic), class B member 2, pseudo gene (HSP90AB2P) | NR_ 003132.1 | |
| NR_ 003133 | 30208.2 | 357.1 | 84.6 | Homo sapiens guanylate binding protein 1, interferon-inducible pseudogene 1 (GBP1P1), non-coding RNA | NR_ 003133.2 | |
| BX649144 | 12117.1 | 153.4 | 79.0 | Tubulin tyrosine ligase (TTL) | NM_153712.4 | NP_714923.1 |
| DERP7 | 7428.7 | 101.5 | 73.2 | transmembrane protein 45A (TMEM45A) | NM_ 018004.1 | NP_ 060474.1 |
| MGC4836 | 5461.5 | 38.3 | 142.7 | Homo sapiens similar to hypothetical protein (L1H 3 region) | BC036758.2 | AAH36758.1 |
| BC037952 | 2188.6 | 36.4 | 60.1 | cDNA clone | BC037952.1 | |
| AK056558 | 1960.9 | 18.6 | 105.2 | cDNA clone | AK056558.1 | |
| NM_ 001001704 | 1703.9 | 68.4 | 24.9 | FLJ44796 hypothetical | NM_ 001001704.1 | NP_001001704.1 |
| ODF2L | 1213.9 | 22.0 | 55.2 | outer dense fiber of sperm tails 2-like (ODF2L) | NM_ 020729.2 | NP_ 065780.2 |
| | | | | | NM_ 001007022.2 | NP_ 001007023.2 |
| | | | | | NM_ 001184765.1 | NP_ 001171694.1 |
| | | | | | NM_ 001184766.1 | NP_ 001171695.1 |
| BC041426 | 1020.8 | 43.0 | 23.7 | C12orf55 chromosome 12 open reading frame 55 (C12orf55) | XM_ 001715090.3 | XP_001715142.3 |
| REXO1L1 | 1013.4 | 9.9 | 102.2 | RNA exonuclease 1 homolog (S. cerevisiae)-like 1 (REXO1 L1) | NM_ 172239.4 | NP_758439.4 |
| AK026100 | 977.9 | 35.8 | 27.3 | FLJ22447 hypothetical LOC400221 (FLJ22447) | NR_ 039985.1 | |
| AK026825 | 823.9 | 13.5 | 61.1 | transmembrane protein 212 (TMEM212) | NM_ 001164436.1 | NP_ 001157908.1 |
| KENAE1 | 793.7 | 36.9 | 21.5 | Homo sapiens mRNA for Kernel (AB024691) | AB024691.1 | BAC57450.1 |
| HESRG | 790.4 | 7.6 | 104.5 | ESRG hypothetical LOC790952 (ESRG) | NR_ 027122.1 | |
| AK095450 | 764.2 | 35.5 | 21.5 | LOC285540 hypothetical LOC285540 | NR_ 037934.1 | |
| FLJ36492 | 733.9 | 20.9 | 35.1 | CCR4-NOT transcription complex, subunit 1 (CNOT1) | NM_ 016284.3 | NP_ 057368.3 |
| | | | | | NM_ 206999.1 | NP_ 996882.1 |
| AK124194 | 688.2 | 13.4 | 51.2 | FLJ42200 protein | AK124194.1 | BAC85800.1 |
| AK096196 | 619.2 | 11.0 | 56.0 | hypothetical LOC100129434 | XR_ 109938.1 | |
| AK131313 | 580.8 | 11.7 | 49.5 | Zinc finger protein 91 pseudogene (LOC441666) | NR_ 024380.1 | |
| FLJ11292 | 547.0 | 22.8 | 24.0 | hypothetical protein FLJ11292 | NM_ 018382.1 | NP_060852.1 |
| CCDC122 | 541.3 | 13.1 | 41.5 | coiled-coil domain containing 122 (CCDC122) | NM_ 144974.3 | NP_ 659411.2 |
| BC070093 | 522.8 | 4.3 | 121.1 | cDNA clone | BC070093.1 | |

### Example 5. Validation using oligonucleotide branched DNA hybridization assay

Candidate SCGs identified from Helicos RNA sequencing criteria as described above (and listed in Table 7) were further evaluated using Affymetrix QUANTIGENE probes. Total RNA from 4 primary pancreatic ductal adenocarcinomas (PDAC) were analysed using the QUANTIGENE Plex RNA assay. The results are shown below (Table 8).

| **Table 8** | | | |
|---|---|---|---|
| **Human Gene Name** | **PDAC AVG Signal** | **Cell Line AVG Signal** | **RATIO PDAC/Cell Line** |
| HSP90BB | 36.17 | 6.22 | 5.81 |
| KENAE1 | 0.19 | 0.16 | 1.16 |
| AK056558 | 20.12 | 5.19 | 3.88 |
| MGC4836 | 1.01 | 0.90 | 1.11 |
| BC037952 | 7.78 | 2.22 | 3.50 |
| BC070093 | 0.14 | 0.01 | 18.40 |
| BC041426 | 0.04 | 0.00 | 23.22 |
| CCDC122 | 0.06 | 0.06 | 0.97 |
| FLJ36492 | 1.22 | 1.14 | 1.07 |
| HESRG | 1.22 | 0.11 | 11.16 |
| FLJ11292 | 0.04 | 0.00 | 78.08 |
| AK026100 | 0.04 | 0.00 | 9.04 |
| AK124194 | 0.03 | 0.00 | 9.28 |
| NM_001001704 | 1.36 | 0.24 | 5.61 |
| AK096196 | 10.87 | 0.32 | 33.89 |
| AK095450 | 0.32 | 0.01 | 24.64 |
| AK131313 | 1.04 | 0.58 | 1.81 |
| NR_003133 | 0.21 | 0.01 | 32.70 |
| ODF2L | 0.55 | 0.12 | 4.52 |
| REXO1L1 | 0.14 | 0.00 | 88.12 |
| AK026825 | 0.05 | 0.01 | 4.37 |
| DERP7 | 0.12 | 0.10 | 1.25 |
| BX649144 | 0.22 | 0.19 | 1.20 |

Based on this data, Affymetrix VIEWRNA probes were developed for testing in formalin fixed paraffin embedded (FFPE) primary tumor specimens for HSP90BB and AK096196. These probes were tested using the RNA in situ hybridization (RNA-ISH) assay at MGH on FFPE material. Positive staining was seen on human cancer subcutaneous xenografts made in Nu/Nu mice using colon cancer cell line HCT-116. HSP90BB was further tested in primary human PDAC specimens from the MGH; the results, shown in Fig. 5, demonstrated abundant and specific staining of epithelial ductal carcinoma cells.

## Claims

1. An in vitro method of detecting the presence of pancreatic cancer in a subject, the method comprising:
determining an expression level of HSP90BB (heat shock protein 90kDa alpha (cytosolic), class B member 2, pseudogene (HSP90AB2P)) in a sample comprising a test cell from the subject to obtain a test value; and
comparing the test value to a reference value,
wherein a test value that is significantly above the reference value indicates that the subject has pancreatic cancer.

2. The method of claim 1, wherein the reference level is a level of HSP90BB in a normal cell.

3. The method of claim 2, wherein the normal cell is a cell of the same type as the test cell in the same subject or in a subject who does not have cancer.

4. The method of claim 1, wherein the sample is known or suspected to comprise tumor cells, optionally wherein the sample is a blood sample known or suspected of comprising circulating tumor cells (CTCs), or a biopsy sample known or suspected of comprising tumor cells.

5. An in vitro method of evaluating the efficacy of a treatment for pancreatic cancer in a subject, the method comprising:
determining a level of HSP90BB in a first sample from the subject to obtain a first value;
determining a level of HSP90BB in a subsequent sample obtained from the subject at a later time after the treatment, to obtain a treatment value; and
comparing the first value to the treatment value,
wherein a treatment value that is below the first value indicates that the treatment is effective.

6. The method of claim 5, wherein the first and second samples are known or suspected to comprise tumor cells, optionally wherein the samples are blood samples known or suspected of comprising circulating tumor cells (CTCs), or biopsy samples known or suspected of comprising tumor cells.

7. The method of claim 5, wherein the treatment is a surgical intervention, chemotherapy, radiation therapy, or a combination thereof.

8. The method of any of the preceding claims, wherein determining a level of HSP90BB comprises determining a level of a transcript.

9. The method of claim 8, wherein determining a level of a transcript comprises contacting the sample with an oligonucleotide probe that binds specifically to the transcript, optionally wherein the probe is labeled.

10. The method of claim 9, wherein the method comprises amplifying the transcript.

11. The method of claim 8, wherein determining a level of a transcript comprises performing RNA sequencing.

12. The method of any of the preceding claims, wherein the subject is a human.

## Patentansprüche

1. In vitro-Verfahren zum Nachweisen des Vorliegens von Pankreaskrebs bei einem Individuum, wobei das Verfahren umfasst:
Bestimmen eines Expressionsniveaus von HSP90BB (Hitzeschockprotein 90 kDa alpha (zytosolisch), Klasse B Mitglied 2, Pseudogen (HSP90AB2P)) in einer Probe, umfassend eine Testzelle von dem Individuum, um einen Testwert zu erhalten; und
Vergleichen des Testwerts mit einem Referenzwert,
worin ein Testwert, der signifikant über dem Referenzwert ist, anzeigt, dass das Individuum Pankreaskrebs hat.

2. Verfahren nach Anspruch 1, worin das Referenzniveau ein Niveau von HSP90BB in einer normalen Zelle ist.

3. Verfahren nach Anspruch 2, worin die normale Zelle eine Zelle des gleichen Typs wie die Testzelle in dem gleichen Individuum oder in einem Individuum, das keinen Krebs hat, ist.

4. Verfahren nach Anspruch 1, worin bekannt ist oder vermutet wird, dass die Probe Tumorzellen umfasst, optional, worin die Probe eine Blutprobe ist, von der bekannt ist oder vermutet wird, dass sie zirkulierende Tumorzellen (ZTZ) umfasst, oder eine Biopsieprobe ist, von der bekannt ist oder vermutet wird, dass sie Tumorzellen umfasst.

5. In vitro-Verfahren zum Beurteilen der Wirksamkeit einer Behandlung von Pankreaskrebs eines Individuums, wobei das Verfahren umfasst:
Bestimmen eines Niveaus von HSP90BB in einer ersten Probe von dem Individuum, zum erhalten eines ersten Werts;
Bestimmen eines Niveaus von HSP90BB in einer nachfolgenden Probe, die von dem Individuum zu einem späteren Zeitpunkt nach der Behandlung erhalten wurde, um einen Behandlungswert zu erhalten; und
Vergleichen des ersten Werts mit dem Behandlungswert,
worin ein Behandlungswert, der unter dem ersten Wert liegt, anzeigt, dass die Behandlung wirkungsvoll ist.

6. Verfahren nach Anspruch 5, worin von den ersten und zweiten Proben bekannt ist oder angenommen wird, dass sie Tumorzellen umfassen, optional, worin die Proben Blutproben sind, von denen bekannt ist oder vermutet wird, dass sie zirkulierende Tumorzellen (ZTZ) umfassen, oder Biopsieproben sind, von denen bekannt ist oder vermutet wird, dass sie Tumorzellen umfassen.

7. Verfahren nach Anspruch 5, worin die Behandlung ein chirurgischer Eingriff, Chemotherapie, Strahlentherapie oder eine Kombination davon ist.

8. Verfahren nach einem der vorhergehenden Ansprüche, worin Bestimmen eines Niveaus von HSP90BB das Bestimmen eines Niveaus eines Transkripts umfasst.

9. Verfahren nach Anspruch 8, worin Bestimmen eines Niveaus eines Transkripts das In-Kontakt-Bringen der Probe mit einer Oligonukleotidsonde, die spezifisch an des Transkript bindet, worin optional die Sonde markiert ist, umfasst.

10. Verfahren nach Anspruch 9, worin das Verfahren Amplifizieren des Transkripts umfasst.

11. Verfahren nach Anspruch 8, worin das Bestimmen eines Niveaus eines Transkripts das Durchführen von RNA-Sequenzierung umfasst.

12. Verfahren nach einem der vorhergehenden Ansprüche, worin das Individuum ein Mensch ist.

## Revendications

1. Procédé in vitro de détection de la présence d'un cancer du pancréas chez un sujet, le procédé comprenant le fait :
de déterminer un niveau d'expression de HSP90BB (protéine de choc thermique 90kDa alpha (cytosolique), membre 2 de classe B, pseudogène (HSP90AB2P)) dans un échantillon comprenant une cellule de test provenant du sujet pour obtenir une valeur de test ; et
de comparer la valeur de test à une valeur de référence,
dans lequel une valeur de test qui est significativement supérieure à la valeur de référence indique que le sujet a un cancer du pancréas.

2. Procédé de la revendication 1, dans lequel le niveau de référence est un niveau de HSP90BB dans une cellule normale.

3. Procédé de la revendication 2, dans lequel la cellule normale est une cellule du même type que la cellule de test chez le même sujet ou chez un sujet qui n'a pas de cancer.

4. Procédé de la revendication 1, dans lequel l'échantillon est connu pour ou suspecté de contenir des cellules tumorales, facultativement dans lequel l'échantillon est un échantillon de sang connu pour ou suspecté de contenir des cellules tumorales circulantes (CTC), ou un échantillon de biopsie connu pour ou suspecté de contenir des cellules tumorales.

5. Procédé in vitro d'évaluation de l'efficacité d'un traitement du cancer du pancréas chez un sujet, le procédé comprenant le fait :
de déterminer un niveau de HSP90BB dans un premier échantillon provenant du sujet pour obtenir une première valeur ;
de déterminer un niveau de HSP90BB dans un échantillon suivant obtenu à partir du sujet ultérieurement après le traitement, pour obtenir une valeur de traitement ; et
de comparer la première valeur à la valeur de traitement,
dans lequel une valeur de traitement qui est inférieure à la première valeur indique que le traitement est efficace.

6. Procédé de la revendication 5, dans lequel les premier et deuxième échantillons sont connus pour ou suspectés de contenir des cellules tumorales, facultativement dans lequel les échantillons sont des échantillons de sang connus pour ou suspectés de contenir des cellules tumorales circulantes (CTC), ou des échantillons de biopsie connus pour ou suspectés de contenir des cellules tumorales.

7. Procédé de la revendication 5, dans lequel le traitement est une intervention chirurgicale, une chimiothérapie, une radiothérapie, ou une combinaison de celles-ci.

8. Procédé de l'une des revendications précédentes, dans lequel la détermination d'un niveau de HSP90BB comprend la détermination d'un niveau d'un produit de la transcription.

9. Procédé de la revendication 8, dans lequel la détermination d'un niveau d'un produit de la transcription comprend la mise en contact de l'échantillon avec une sonde oligonucléotidique qui se lie spécifiquement au produit de la transcription, facultativement dans lequel la sonde est marquée.

10. Procédé de la revendication 9, dans lequel le procédé comprend l'amplification du produit de la transcription.

11. Procédé de la revendication 8, dans lequel la détermination d'un niveau d'un produit de la transcription comprend la réalisation d'un séquençage d'ARN.

12. Procédé de l'une des revendications précédentes, dans lequel le sujet est un être humain.
